# EUROPEAN PATENT SPECIFICATION

(11) **EP 2 841 117 B1**
(45) Date of publication and mention of the grant of the patent: **27.11.2019**
(21) Application number: 13724440.6
(22) Date of filing: 26.04.2013
(51) Int. Cl.: A61L 27/28, A61L 27/40, A61L 27/54, A61L 31/08, A61L 31/12, A61L 31/16

(54) **COMPOSITIONS AND METHODS FOR COATING IMPLANT SURFACES TO INHIBIT SURGICAL INFECTIONS**
ZUSAMMENSETZUNGEN UND VERFAHREN ZUR BESCHICHTUNG VON IMPLANTATOBERFLÄCHEN ZUR VERHINDERUNG CHIRURGISCHER INFEKTIONEN
COMPOSITIONS ET PROCÉDÉS DE REVÊTEMENT DE SURFACES D'IMPLANTS POUR INHIBER LES INFECTIONS CHIRURGICALES

(30) Priority: 27.04.2012 US 201261639314 P; 15.03.2013 US 201361799929 P
(43) Date of publication of application: 04.03.2015
(73) Proprietor: Biomet Manufacturing, LLC, Warsaw, IN 46582 (US)
(72) Inventor: TROXEL, Karen S., Warsaw, Indiana 46580 (US); PONTICIELLO, Michael, Mission Viejo, California 92691 (US); HERSHBERGER, Troy W., Winona Lake, Indiana 46590 (US)
(74) Representative: Manitz Finsterwald Patent- und Rechtsanwaltspartnerschaft mbB
(86) International application number: PCT/US2013/038460
(87) International publication number: WO 2013/163577

(56) References cited:
- EP-A1- 1 683 531
- US-A- 5 755 788

## Description

### INTRODUCTION

The present technology relates to compositions for coating orthopedic implants with an infection-inhibiting agent, as well as methods for making such compositions and methods and devices for intra-operative coating of orthopedic implants prior to implantation.

Orthopedic implants are implantable medical devices used replace, augment or repair bone, such as to replace diseased articulating joints (such as knees, hips and elbows), stabilize the skeleton where it has been destabilized by trauma (such as fractures), or to correct alignment. These implants are manufactured most commonly with plastics, polymers, ceramics, steel, stainless steel, metals and alloys.

However, as foreign bodies, the surfaces of orthopedic devices implanted into the body provide a physical platform for bacteria to attach and grow. Due to the rapid growth rate and presence of virulence factors, bacteria are able to establish infections within days of the surgical procedure causing loss of implant fixation, local tissue inflammation, and local tissue necrosis due to sepsis. The most common organisms causing these infectious complications are *Staphylococcus epidermidis*, *Staphylococcus aureus* and *Pseudomonas sp.* when the injuries are related to trauma or battlefield injuries. In the case of orthopedic procedures, *Staphylococcus epidermidis*, *Staphylococcus aureus* account for almost 70-80% of all infectious organisms, with *Staphylococcus epidermidis* being the most common organism.

Such device-related infections may not be successfully treated by systemic antibiotics alone. Generally, successful eradication of the infection requires removal of the hardware, followed by local antibiotic therapy with a temporary tissue spacer combined with several weeks of systemic antibiotic therapy. After the infection is cleared, a permanent device is implanted in yet another surgical procedure. Thus, the treatment of infected orthopedic devices may involve extensive hospitalization, multiple surgeries, and is associated with significant morbidity due to limited motility in addition to the surgical procedures.

A considerable amount of attention and study has been directed toward preventing such infections. For example, there has been much research of coatings and surface treatments that would either inhibit bacterial attachment or inhibit bacterial growth as a means to prevent device infection. Some of these technologies focus on making the surface "non-stick" for the bacteria; other technologies involve a coating carrier matrix, typically a polymer that delivers an antimicrobial agent such as antibiotic, antiseptic or silver ion to the surface.

For example, EP 1 683 531 A1 discloses a coating for implants consisting of a hydrophobic matrix material, a hydrophobic fatty acid-based additive and an antibiotic. US 5,775,788 A relates to infection-resistant implants having bound to the surfaces thereof freeze-dried liposomes enveloping an antibiotic and capable of rapid reconstitution at the moment of surgery.

Nevertheless, many approaches employed in the art are deficient, for one or more reasons. For example, application of an antimicrobial coating to the surface of an orthopedic device prior to packaging and delivery to the hospital would be convenient for the surgeon who is implanting the device in a surgical procedure. Unfortunately, the application of an antimicrobial coating by the orthopedic device manufacturer prior to packaging adds an unacceptably high cost to the implantable device. As a result few, if any, pre-coated devices are commercially available. Furthermore, stability of formulations may be a concern because the shelf life of the antimicrobial formulation is typically much shorter than the shelf life of the medical device itself.

Alternatively, methods for application of antimicrobial agents to an orthopedic device surface in the operating room are either not expected to be effectively antimicrobial, or are unpractical to execute in the operating theater. Some approaches employ coatings that are difficult to apply, may result in uneven and inadequate delivery of the anti-infective, offer ineffective levels or duration of antimicrobial protection, or that do not survive the rigors of surgical implantation procedures.

For example, one way to treat device surfaces with antimicrobial agents in the operating room might be to apply an antibiotic solution to the surface by soaking the device or spraying the device in rehydrated antibiotic preparations formulated for intravenous administration. However, the amount of solution deposited is small because the solution runs or drips off leaving only a very thin film on the surface. Thus, while this method is simple, it is not effective.

Alternatively, coatings with polymer or lipid matrix carriers might be applied in the operating room with spraying equipment. A carrier might allow buildup of sufficient coating thickness to provide an effective amount of antimicrobial agent. However, the carrier material (polymer, lipid, wax, protein) and the active antimicrobial agent would need to be in a solution in order to apply it to the surface of the implant by spraying or dripping or dipping. If the solution is aqueous, it will take a long time to dry, and this is unacceptable in the operating room. Alternatively, if the solution is a non-aqueous organic solvent, the drying time would be shorter but the operating room personnel would be exposed to the evaporated solvent vapors. This would add unacceptable health hazard risk to the operating room personnel and would not be an attractive technology for that reason. Even if the spraying operation is enclosed in a closed chamber, thereby containing the organic solvent fumes or protecting the implant while the water dries, there would be the inconvenience of introducing yet another piece of equipment into the operating room. Overall, the concept of spray-coating an implant in the operating room would be unacceptable because it requires significant time of operating room personnel, and/or requires unacceptable organic solvents or expensive additional equipment.

Accordingly, health care providers have few, if any, clinically viable methods for coating implants to prevent surgical infections in cases where patient-related and procedure-related risk factors for surgical site infection have been identified. Thus, there remains a need to develop novel biocompatible coatings for medical devices that contain infection-inhibiting agents. There is a particular need for novel coating systems that can apply an infection-inhibiting coating to an orthopedic implant surface in the operating room, which is safe, easy to use, and having sufficient flexibility for the surgeon to determine which implants require an antimicrobial coating based upon patient risk factors and procedural risk factors.

### SUMMARY

The present technology provides infection-inhibiting compositions suitable for coating surfaces of implantable medical implants, including compositions and devices for coating medical devices in the operating room prior to implantation in a patient. The compositions have a waxy matrix comprising phosphatidylcholine, wherein the matrix is operable to deposit the anti-infective when rubbed on a surface of a device.

The compositions comprise an antimicrobial agent. Thus, the present technology also provides compositions comprising a safe and effective amount of an antimicrobial agent and a waxy carrier operable to deposit the antimicrobial agent when rubbed on a surface of the device. Antimicrobial agents among those useful herein in include antibiotics, antiseptics, antiadhesion agents, and combinations thereof. For example, antimicrobial agent may be selected from the group consisting of rifampin, fosfomycin, tetracyclines, aminoglycosides, quinolones, glycopeptides, antimicrobial peptides, disinfectants, antimicrobial metal ions (such as silver), and mixtures thereof.

Also disclosed are compositions which are in a solid "stick" form, having a waxy consistency that holds its shape under gentle pressure used to deposit lipid onto the surface of the implant to be treated. Also disclosed are application devices that support the stick during application and deploy the stick as lipid is deposited on the surface. The compositions may be applied to the medical device using an infection-inhibiting coating system comprising a) a composition comprising a safe and effective amount of an infection-inhibiting agent, and a waxy carrier operable to deposit the anti-infective when rubbed on a surface of the device; and b) an applicator containing the infection-inhibiting composition.

The present technology also provides orthopedic devices for inhibiting infection at the site of implantation of the orthopedic device in a human or animal subject, obtained by a method comprising rubbing a surface of the device, prior to implantation, with an infection-inhibiting composition comprising phosphatidylcholine and having a waxy matrix comprising an infection-inhibiting material selected from the group consisting of a lipid, an antimicrobial agent, and mixtures thereof, wherein a thin layer of the infection-inhibiting material is deposited on the surface of the device. Medical implants which may be treated in the methods of this technology include orthopedic implants. Such implants include hip implants, knee implants, elbow implants, prosthetic frames, bone prostheses, small joint prostheses, and fixation devices. The surfaces of the implants to be coated may be made of any physiologically suitable material, such as metals, ceramic, or polymers.

Medical devices having a coating of the present technology may be made by methods comprising rubbing a composition of the technology on a surface of the medical device, wherein a thin layer of lipid is coated on the surface. Optionally, in embodiments wherein the composition comprises an antimicrobial agent, a safe and effective amount of the antimicrobial agent is also deposited. The articles thus produced by the methods disclosed herein include hip implants, knee implants, elbow implants, prosthetic frames, bone prostheses, small joint prostheses, and fixation devices.

### DRAWINGS

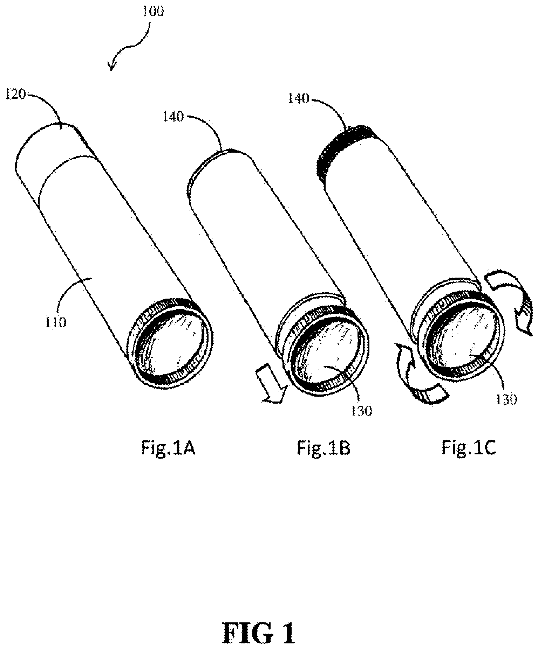

Figure 1 is a diagrammatic illustration of an infection-inhibiting delivery system suitable for coating an implant.

It should be noted that the figures set forth herein are intended to exemplify the general characteristics of materials and methods among those of the present technology, for the purpose of the description of certain embodiments. These figures may not precisely reflect the characteristics of any given embodiment, and are not necessarily intended to define or limit specific embodiments within the scope of this technology.

### DETAILED DESCRIPTION

The following description of technology is merely exemplary in nature of the subject matter, manufacture and use of one or more inventions, and is not intended to limit the scope, application, or uses of any specific invention claimed in this application or in such other applications as may be filed claiming priority to this application, or patents issuing therefrom. A non-limiting discussion of terms and phrases intended to aid understanding of the present technology is provided at the end of this Detailed Description.

The present technology provides compositions and orthopedic devices with an infection-inhibiting material comprising phosphatidylcholine.

### Waxy Matrix

The waxy matrices of the compositions of the present technology are operable to deposit an infection-inhibiting material when rubbed on a surface of a device. The matrix can be formulated into a stick that holds its shape under gentle pressure used to transfer the matrix onto a surface of an implant. In particular, as discussed further below, the composition is solid and does not readily flow (except under force).

Waxy matrices useful preferably herein include those that 1) leave a thin layer on the surface of an implant when rubbed over its surface; 2) dissolve, resorb or otherwise dissipate from the implant surface after implantation; and 3) do not illicit any adverse tissue reactions at the locus of the implant or after absorption into the blood or lymphatic system. Further, in compositions comprising an optional antimicrobial agent, the waxy matrices are also mixable with antimicrobial agent at temperatures below temperatures that lead to thermal destruction of the antimicrobial agents. Preferably the waxy matrix does not interfere with tissue adhesion to the surface of the device, especially for implants such as non-cemented joint replacement components or dental implants. Preferably the compositions of the present technology are substantially free of solvents.

In various embodiments, the matrix comprises phosphatidylcholine as an infection-inhibiting material. Without limiting their mechanism, function, or utility, such compositions are biocompatible, do not interfere with bone repair, dissolve readily in vivo, and are metabolized by the body leaving nontoxic degradation products. Compositions comprise phosphatidylcholine that either naturally have a waxy consistency or have been formulated with other materials, such as other lipids and viscosity modifiers, to produce a waxy consistency.

Lipids useful herein include naturally occurring compounds that generally are defined as fatty acids and their derivatives, and biosynthetically or functionally related compounds. Classes of lipid structures include fatty acids, simple glycerolipids including triacylglyerols and diacylglycerols, and glycerophospholipids (phospholipids). The lipid-based composition may comprise sufficient long-chain diacylglycerides or triacylglycerides, either saturated or unsaturated, to provide stiffness to the formulation so that it is solid at room temperature and has a waxy texture. Examples of saturated fatty acids include caproic acid, enanthic acid, caprylic acid, pelargonic acid, capric acid, undecylic acid, lauric acid, tridecylic acid, myristic acid, pentadecylic acid, palmitic acid, margaric acid, stearic acid, nonadecylic acid, arachidic acid, heneicosylic acid, behenic acid, tricosylic acid, lignoceric acid, pentacoylic acid, cerotic acid, heptacosylic acid, montanic acid, nonacosylic acid, melissic acid, nenatriacontylic acid, lacceroic acid, psyllic acid, geddic acid, ceroplastic acid and hexatriacontylic acid. Examples of unsaturated fatty acids include myristoleic acid, palmitoleic acid, sapienic acid, oleic acid, elaidic acid, vaccenic acid, linoleic acid, linoelaidic acid, □-linolenic acid, arachidonic acid, eicosapentaenoic acid, erucic acid and docosahexaenoic acid. Furthermore, the unsaturated fatty acids may be polyunsaturated. In addition to the lipids that are solid at room temperature, other lipids that are liquid at room temperature may be added to soften the hardness of the solid lipids.

In some embodiments, the lipid matrix comprises (by weight % of the matrix) 50% or more, 60% or more, 70% or more, 80% or more, 90% or more, or 95% or more phospholipid such as phosphatidylcholine. In some embodiments, the lipid matrix consists essentially of phospholipids. Phospholipids are amphipathic molecules that are characterized by a hydrophilic head, which consists of a polar group, a phosphate and glycerol, and two hydrophobic fatty acid tails, which may be saturated, unsaturated, polyunsaturated or combinations thereof. The fatty acids of synthetic phospholipids may be medium-chain fatty acids with 6-12 carbons, long-chain fatty acids with more than 12 carbons or very long-chain fatty acids with more than 22 carbons. In various embodiments, the fatty acid tails are either 16 or 18 carbons long, wherein the 18-carbon chains are predominantly unsaturated.

It is also disclosed that the phospholipid can be a mixture of phospholipids, for example, a mixture of phosphatidylcholine, phosphatidylethanolamine and phosphatidylinositol as is present in lecithin. Lecithin may be isolated from plant (such as soy bean) and animal tissues. Commercially available lecithin useful herein includes Phosal® 53 MCT, (from Lipoid, GmbH, Köln, Germany) which comprises at least about 53% phosphatidylcholine, up to 6% lysophosphatidylcholine and from 3% to 6% ethanol.

Phosphatidylcholine is a preferred phospholipid. Phosphatidylcholines are the most abundant form of lipid component in cellular membranes. They are amphipathic, having both hydrophobic sections in the fatty acid tails and hydrophilic portions in the phosphate and choline groups. In naturally occurring phosphatidylcholines the fatty acid tails are either 16- or 18-carbons long and the 18-carbon chains are predominantly unsaturated. Such a preparation is commercially available as Phospholipon® 90G (from Lipoid GmbH, Köln, Germany) and comprises at least 94% phosphatidylcholine, up to 4.0% lysophosphatidylcholine and up to 0.3% tocopherol. Phosphatidylcholine derived from soybeans and purified is a slightly yellow, waxy solid. Purified phosphatidylcholine such as Phospholipon 90G makes an excellent coating stick and it is also an excellent carrier for optional additives such as bioactive antimicrobial agents. Purified phosphatidylcholine from either soybean or from eggs is available from several commercial sources; it is used in the food processing industry. Other glycerophospholipids such as phosphatidylserine, phosphatidylinositol, and phosphatidylethanolamine may be used.

Synthetic phosphatidylcholines are also available from several sources and with different lengths of fatty acid chains and different degrees of saturation. These are readily available commercially and are used in drug delivery formulation. Also, naturally derived purified phosphatidylcholine can be hydrogenated to fully saturate the fatty acid chains, producing a solid that is a white powder and no longer has a waxy consistency. The hydrogenated phosphatidylcholine and the synthetic phosphatidylcholines can be combined with other lipids to create a stick product with the physical characteristics necessary to achieve the proper hardness and the ability to transfer easily and smoothly to the surface of a device. For instance, hydrogenated phosphatidylcholine can be added to natural unsaturated phosphatidylcholine in order to increase the stiffness of the stick product. Alternatively, lipids that are liquid at room temperature such as medium chain triacylglycerols found in corn oil, olive oil, palm oil, sunflower oil, or rapeseed oil for instance, or unsaturated simple fatty acids such as oleic or linolenic acid, can be used to soften a stiffer lipid such as hydrogenated phosphatidylcholine or fully saturated triacylglycerols such as tristearate, trimyristate, or tripalmitate in order to produce a coating stick product with the proper hardness and ease of application. If an additive such as an antimicrobial agent needs to be pre-mixed with a liquid lipid mixture such as Phosal 53MCT prior to addition to the matrix in order to utilize a mechanical mixing process, then a stiffer lipid such as a fully saturated (hydrogenated) phosphatidylcholine can be added to the coating stick formulation in order to counteract the softening effects of the liquid lipid component.

For application to the surfaces of orthopedic implants that require biologic fixation, purified soy derived phosphatidylcholine is a particularly preferred lipid because it dissolves readily and will not interfere with biologic fixation, and is biocompatible and compatible with bone repair. The amphipathic nature and the unsaturated fatty acid components allow the phosphatidylcholine to disperse rapidly in aqueous environments such as in vivo. Fully saturated triacylglyerols and saturated fatty acids are not readily soluble in water, hindering dispersion in vivo. This may limit their application to the surface of implants that do not require bone or tissue attachment, such as fracture hardware, plates, screws, intramedullary nails. Accordingly, except as used to modify the rheology of compositions as discussed above, the compositions of the present technology are preferably substantially free (containing less than 5%, preferably less than 1%, or preferably 0%) of fully saturated triacylglyerols and saturated fatty acids.

As discussed above, the compositions of this technology have a rheology that is a non-flowable solid at ambient conditions, preferably also at body temperature. The compositions are preferably not "putty-like" or malleable with moderate pressure (by hand) under ambient conditions. The physical properties of a composition renders it operable to transfer material from the composition, in particular an infection-inhibiting material, onto a surface of an implant by rubbing the composition over the surface with moderate pressure, by hand. Preferably the resulting coating is smooth, even, adherent, not flaky, and easily applied. The coating may transfer to tissue at the site of implantation, while remaining sufficiently pliable and adherent that it does not come off of the surface of the implant in flakes. Such compositions may have a viscosity equivalent to crayons, lipstick, lip balm, and similar compositions known in the art for the delivery of pigments, cosmetic and pharmaceutical actives to tissues and other surfaces.

The composition preferably has a Cone Penetration Hardness of from at least 1.5 lbf (6.67 N) to 15 lbf (66.7 N) in a cone penetration test, as described below. "Cone Penetration Hardness" is defined as the peak force experienced in moving a penetration cone through the composition for a distance of 5 mm at a rate of 1 mm/second. The cone has a maximum diameter of 6.25 mm and widens from a point to a maximum diameter over a length of 6 mm at an angle of 27.5 degrees. In various embodiments, the hardness is at least 3 lbf (13.34 N), at least 5 lbf (22.24 N), or at least 10 lbf (44.48 N). The hardness may be less than 10 lbf (44.48 N), in some embodiments.

### Infection-Inhibiting Material

Compositions of the present technology comprise a safe and effective amount of an infection-inhibiting material in a waxy carrier operable to deposit the anti-infective when rubbed on a surface of the device. Such a "safe and effective amount" is sufficient to have the desired infection-inhibiting effect in the human or lower animal subject, without undue adverse side effects (such as toxicity, irritation, or allergic response), commensurate with a reasonable benefit/risk ratio when used in the manner of this technology. The specific safe and effective amount of the infection-inhibiting material will vary with such factors as the particular surgical procedure, the surface characteristics of the implant device (such as material, texture and contours), the condition and characteristics of the tissue into which the device is implanted the physical condition of the patient, the nature of concurrent therapy (if any), the specific infection-inhibiting material used, and other materials (if any) present in the composition matrix. The infection-inhibiting effect preferably substantially reduces the number of microbes on the treated surface of the implant after implantation of the device relative to the number of microbes that would be present on the implant without coating.

As referred to herein, an "infection-inhibiting" material inhibits the attachment of one or more microbial organisms (e.g., bacteria, yeast and other fungal organisms) on the surface to which it is applied. Such inhibition of surface attachment prevents development of biofilm-based microbial phases of growth at the site of the implant, thus the prevention of adhesion maintains the microbes in a state bioavailable to the host immune system. In some embodiments, microbial growth is substantially prevented or suppressed, wherein microbes are present (if present) following implantation at a level allowing the immune system of the surgical subject to recognize and neutralize remaining microbes. The infection-inhibiting material reduces the likelihood of device-related infection or surgical site infection.

In orthopedic surgical procedures, microbes ("target microbes") include organisms that are associated with the device or may be otherwise present at the site of the device, which may in bone or surrounding tissues such as skin, blood, muscle, cartilage, and bone. Thus, any microbe that has the potential to enter surreptitiously and colonize at a surgical site or area of orthopedic repair and trauma may be targeted in accordance with the present technology. Target microbes of particular concern are those that colonize the skin of a surgical subject, since these organisms may enter the subject at the site where the orthopedic implant was inserted.

Particularly relevant target microbes include Gram-positive and Gram-negative bacteria, and yeasts. Such organisms include *Klebsiella*, *Enterobacter*, *Acinetobacter*, *Pseudomonas*, *Escherichia*, and *Staphylococcus.* Specific bacteria include *Staphylococcus aureus*, as represented by strain NCTC 8325 and methicillin resistant strains which presently cause significant problems in hospital environments. Further targets are *Staphylococcus epidermidis*, represented by strain NCTC 11047, and yeasts such as *Candida albicans*, represented by strain ATCC 26555. Some of these bacteria are known to produce fibrinogen-binding clumping factors A and B and the fibronectin-binding protein (FnbA), capable of adhering to orthopedic implants and related devices.

As discussed above, the infection-inhibiting composition comprises phosphatidylcholine in whole or in part, the waxy matrix. Thus, in some embodiments, compositions of the present technology consist essentially of an infection-inhibiting phosphatidylcholine, wherein the composition additionally contain an antimicrobial agent (as defined and exemplified below). Without limiting the mechanism, function or utility of the compositions and methods of this technology, in some embodiments the compositions inhibit attachment of organisms to the surface of the implant onto which they are applied, such as by providing a temporary physical barrier to adhesion of bacteria on the surface of the implant. In this regard, it is generally understood that the attachment of bacteria and other microbes to the surface of a device is the first step in the progression of events leading to device-related infection. Subsequent steps involve propagation and creation of a microbial community protected by a polysaccharide extracellular structure known as "biofilm." When attached to the device surface, the bacteria become less available to the host immune system and in biofilm structures they also become less susceptible to the antimicrobial effects of antibiotics. The inhibition of attachment by the compositions of this technology thus keeps the bacteria available to the host immune cells including white blood cells such as polymorphic neutrophils and macrophages.

The anti-adhesion effects of the lipid material may be extended or amplified by the addition of antimicrobial agents that inhibit the growth of the bacteria, inhibit bacterial metabolism, or inhibit the formation of biofilm. Thus, the infection-inhibiting material may be selected from the group consisting of a lipid, antimicrobial agent, and combinations thereof. Specifically, in some embodiments, the present technology provides compositions for application to the surface of an implantable medical device, comprising an antimicrobial agent in a waxy matrix operable to deposit the anti-infective when rubbed on a surface of the device. As noted above, the compositions are preferably resorbable or otherwise dissolvable, such that the optional antimicrobial dissipates from the surface of the device after the device is implanted.

Antimicrobial agents useful in the compositions of the present technology include any compound that has inhibitory activity against the growth of microbes, preferably bacteria as discussed above. Preferably, the anti-infective is selected from the group consisting of antibiotics, antimicrobial peptides, antimicrobial peptide mimetics, disinfectants, antiseptics, antimicrobial metal ions, sugar alcohols, essential oils, salicylic acid, methyl salicylate, nitrous oxide, and mixtures thereof. The amount of antimicrobial agent in the uniform antimicrobial composition is preferably at least 0.1%, at least 1%, at least 5% or at least 10% of the composition. In various embodiments, the concentration is 50% or less, 40% or less, 30% or less, or 20% or less of the composition. For example, the concentration of antimicrobial agent may range from 0.1% to 40%, or from 5% to 35% of the composition.

Suitable antimicrobial agents may have at least one or more of the following properties: 1) the ability to prevent growth and/or replication and/or to kill pathogens which become associated with the orthopedic implant through their ability to bind to blood, muscle and osseous tissue; 2) possessing an acceptable side effect profile, including low toxicity and allergenicity for the intended human or animal subject to be treated; 3) acceptable efficacy at the site of implantation of the coated device, with limited development of microbial resistance; 4) acceptable miscibility or solubility with the carrier; and 5) stability in the coating when applied to the implant.

Antibiotics useful herein include, for example, rifamycins (such as rifampin), fosfomycin, fusidic acid, glycylcyclines, aminoglycosides, quinolones, glycopeptides, bismuth thiols, sulfonamides, trimethoprim, macrolides, oxazolidinones, β-lactams, lincosamides, chloramphenicol, gramicidins, polymyxins, lipodepsipeptides, bacitracins, tetracyclines (such as minocycline), penicillin, ampicillin, cefazolin, clindamycin, erythromycins, levofloxacin, vancomycin, and mixtures thereof.

Tetracycline antibiotics refer to a number of antibiotics of either natural, or semi-synthetic origin, derived from a system of four linearly annealed six-membered rings (1,4,4a,5,5a,6,11,12a-octahydronaphthacene) with a characteristic arrangement of double bonds. The tetracycline antibiotic can include one or more tetracyclines, and/or semi-synthetic tetracyclines such as doxycycline, oxytetracycline, demeclocycline, lymecycline, chlortetracycline, tigecycline and minocycline. A preferred tetracycline is minocycline or minocycline hydrochloride. The amount of tetracycline present in the infection-inhibiting coating can range from 5 µg/cm² to 1000 µg/cm², or from 10 µg/cm² to 800 µg/cm².

Rifamycin class of antibiotics is a subclass of antibiotics from the ansamycin family of antibiotics. The present antibiotic agent or agents can include one or more rifamycin antibiotics from the group rifamycin B, rifampin or rifampicin, rifabutin, rifapentine and rifaximin. Rifampin is commercially available as Rifadin and Rimactane from Sanofi-Aventis U.S. LLC. (Bridgewater, NJ, USA).

Antimicrobial peptides useful herein include, for example, host defense proteins, defensins, magainins, cathetlicidins, protegrins, lantibiotics, nisins, and synthetic mimics of host defense proteins such as cationic steroids. Antiseptics and disinfectants include, for example, chlorhexidine, polyhexanide, triclosan, and iodine-delivering formulas such as betadine or povidone-iodine. Metal ions include various formulations of silver that effectively release silver ions, including silver salts and silver nanoparticles, or copper salts and copper nanoparticles that release copper ions.

Food preservatives that would effectively inhibit microbial attachment or growth include, for example, epsilon polylysine, nisin, and various essential oils including oils from cinnamon, thyme, clove, lemon, lime, orange, and geranium or purified active antimicrobial ingredients from essential oils such as cinnamaldehyde, garnesol, carvacrol, and thymol.

Other antimicrobial agents useful herein include salicylic acid and its metabolite methyl salicylate, and sugar alcohols and polyols (such as xylitol and erythritol). Such sugar alcohols can have antimicrobial properties by preventing bacterial adhesion or bacterial biofilm formation. Polysaccharides, such as chitosan and alginate, are also useful herein.

### Optional Materials

Compositions of the present technology may comprise other materials that (for example) alter the physical characteristics of the compositions or provide therapeutic benefits. Examples include antioxidants, colorants, viscosity modifying agents, and therapeutic actives. For example, polysaccharides, such as carboxymethylcellulose, can be added to improve handling or physical properties. Antioxidants, such as vitamins E and/or C (as tocopherol acetate and ascorbic acid for instance), may also be present. The compositions may also comprise optional active materials, such as small molecule drugs, such as anesthetics (such as bupivacaine) to manage pain, or therapeutic actives including bone and tissue growth promoters and antiinflammatories. Therapeutic actives among those useful herein include bisphosphonates, insulin mimetics (such as vanadium compounds, including vanadyl acetylacetonate), growth factors, and cytokines.

### Methods of Manufacturing

The compositions of the present technology may be made by any suitable process for making lipid compositions, including methods among those known in the art for forming soft solid lipid-containing compositions. In various methods, methods comprise cold forming the composition into a final product form (e.g., a stick). In some embodiments, the forming may be conducted using moderate heat, at a temperature below which the lipid material will degrade.

Mechanical grinding and kneading may be used to make a cohesive composition in a final desired form, e.g., a stick. Mechanical grinding can be performed on an industrial scale by using compounding extruders, such as co-rotating twin screw extruders. Such twin screw extruders are used in the pharmaceutical industry for granulating and compounding, and in the food processing industry for kneading. The screws of the extruders may be either co-rotating or counter-rotating. Additives, such as antimicrobial materials, antioxidants, and other materials, as discussed above, can be added to a hopper either as powders or premixed as a slurry or solution with a liquid lipid, such as Phosal 53MCT or other lipid formulation that is liquid at room temperature. Moderate heat, such as limited exposure to heat at a temperature of from 40° to 80 °C, may aid the mixing process. The mixed and extruded formulations can be cold pressed, or pressed with mild heat, into a desired shape, for example, a cylindrical stick-form, to insert into a dispensing apparatus, as discussed below. If natural phosphatidylcholine or hydrogenated phosphatidylcholine is used, heat is preferably limited to mild temperatures because phospholipids are subject to thermal degradation, and so cannot be melted and then cooled in a mold or applicator. Hot melt extrusion is preferably not employed. An advantage provided by using a compounding or kneading extruder is that there is no introduction of an organic solvent that would later need to be removed from the formulation. The mechanical mixing works well with purified natural phosphatidylcholine, which has a waxy texture as is, and needs no additive to modify the physical properties to get a good stiff waxy stick product.

Alternatively, a phosphatidylcholine based matrix carrier and optional additives can be mixed by first dissolving in organic solvents, preferably a biocompatible solvent, to form true solutions, then mixing the solutions together. A "biocompatible solvent" is a solvent that elicits little or no toxic response in a human or other animal subject. Such solvents useful herein include alcohols, diglycerides, triglycerides, glycerols, polyethylene glycols, saturated or unsaturated free fatty acids (including short, medium and long chain fatty acids and mixtures thereof), tocopherols (such as vitamin E, including tocopherol acetate, alpha tocopherol and gamma tocopherol), and mixtures thereof. The solvents are then removed to leave a completely homogenous lipid-based solid formulation that can be cold molded into a suitable form (e.g., a stick-form) for insertion into an applicator. The solvent removal can be accomplished by either freeze-drying or by spray-drying.

For freeze-drying, the equipment and the solvent are carefully selected so that the eutectic point, or the transition from frozen to sublimation, and therefore the shelf temperature to maintain in the drying phase, is feasible and also that the condenser temperature can be achieved to pull the solvent out of the exhaust. The vacuum pump should be explosion proof. The solvent may alternatively be a mixture comprising organic solvent and water. Appropriate solvents include tertiary butanol, ethanol, isopropanol, acetonitrile and methanol.

Spray-drying may be employed to remove an organic solvent, whereby a solution comprising lipid matrix and additives in the organic solvent is atomized (such as by pressure or ultrasonics) into the top of a tall drying chamber. Very dry gas (such as compressed nitrogen, air, or argon) is also introduced into the top of the chamber. The solvent evaporates into the gas as atomized droplets fall to the bottom of the chamber. The product is collected in the bottom of the chamber and can be removed and cold molded into an appropriate shape before insertion in an applicator. The gas phase is exhausted at the bottom of the chamber as well and sent through a condenser or chiller to remove the solvent for reuse or disposal. Non-limiting examples of solvents used in spray drying operations include methanol, ethanol, toluene, hexane, acetone, ethylacetate, and dichloromethane.

Solvent-based processing can be advantageous if the waxy matrix contains saturated lipids that are normally a powdery solid and that need to be blended with unsaturated or small chain lipids that are liquid at room temperature in order to achieve a waxy texture that will perform well as a coating stick. Saturated lipids that are normally a powder and need such modification include hydrogenated phospholipids and fully saturated triacylglycerols such as trimyristate, tristearate, or tripalmitate. The lipids can be fully mixed with either unsaturated and/or short chain lipids such as unsaturated medium-chain triacylglycerols from corn oil, olive oil, palm oil, sunflower oil, or rapeseed oil for instance, or unsaturated simple fatty acids (oleic acid, linoleic acid). The solvent based process can facilitate the mixing of these types of lipid components.

After packaging the formed product (e.g., a stick, which may be placed into an applicator as discussed below) and wrapping or boxing to maintain a sterile barrier, the product may be terminally sterilized by gamma irradiation or by electron beam sterilization. Alternatively, the product may be prepared and packaged by aseptic processing.

### Devices and Delivery Systems

The present technology provides infection-inhibiting delivery systems comprising a) an infection-inhibiting composition and b) an applicator containing the infection-inhibiting composition. The applicator preferably supports the composition during application and is operable to deploy the composition as material is transferred to the device surface. In some embodiments wherein the composition is in stick form, the applicator comprises a substantially cylindrical tube having an open end and an advancing mechanism at the end of the tube opposite to the open end, wherein the infection-inhibiting composition is contained within the tube, and the advancing mechanism is operable to move the composition along the axis of the tube so as to extend a surface of the infection-inhibiting composition at the open end of the tube.

An exemplary infection-inhibiting delivery system 100 is shown in Figure 1. The delivery system 100 comprises a housing 110, which is a substantially cylindrical tube, and an optional cap 120 for shielding the infection-inhibiting composition 140 when not in use. When in use, as shown in Figure 1B, the base 130 is withdrawn from its secure connection with the housing, as depicted by the large white arrow, and the cap is removed to expose the infection-inhibiting composition 140. Then, as shown in Figure 1C, the advancing mechanism 130 is rotated, as depicted by the curved white arrows, to move the infection-inhibiting composition 140 outwardly from the housing 110. Optionally, rotating the advancing mechanism 130 in the opposite direction urges the infection-inhibiting composition 140 back into the housing 110 until it is completely contained. At this time, the advancing mechanism 130 is then urged toward the housing 110 and snaps into the locked position where no rotation can take place. Such a dispensing device can be used to coat an orthopedic device with an infection-inhibiting composition of the present technology.

### Methods of Coating Implants

Methods for inhibiting the growth of microbes on a surface of a medical implant comprise depositing an infection-inhibiting material on the surface of an implant, by rubbing a composition comprising the infection-inhibiting material on the surface. Accordingly, the present technology provides methods for inhibiting infection at the site of implantation of an orthopedic device in a human or animal subject, comprising rubbing a surface of the device, prior to implantation, with an infection-inhibiting composition comprising phosphatidylcholine and having a waxy matrix comprising an infection-inhibiting material selected from the group consisting of a lipid, an antimicrobial agent, and mixtures thereof, wherein a thin layer of the infection-inhibiting material is deposited on the surface of the device.

Preferably the implant is coated with the infection-inhibiting composition prior to implantation. In particular, the implant may be coated with the infection-inhibiting composition time proximate to the time of implantation. (As used herein, a "proximate" time is any time 24 hours or less before implantation, 4 hours, 2 hours, 1 hour, 30 minutes, 15 minutes, 10 minutes, 2 minutes, 1 minute, or less, before implantation.) Such methods may be considered "intraoperative" wherein the coating is performed by a surgeon or other health care provider as part of the implantation surgery. Such intraoperative methods may offer advantages, such as allowing the health care professional to apply the infection-inhibiting coating in a location, manner, and quantity specifically adapted to the procedure and risks factors for surgical site infection as assessed during the surgery. In various embodiments, the compositions are applied to the surface of the implant in 10 minutes or less, preferably 5 minutes or less.

Implants used in the methods of the present technology include any implant that is at least partially implanted into the body of a subject. An orthopedic implant can include implants that span across the skin layers interfacing with an internal tissue, such as a hard tissue like bone, or a soft tissue like muscle or cartilage, or with another implant. Orthopedic implants useful in the present technology can also include prosthesis parts and accessory components interfacing such prosthesis parts. Generally, the surfaces of the implant are completely or partially implanted into the body of the subject, comprising a metal substrate having one or more surfaces operable to contact a bone tissue or soft tissue when implanted. Orthopedic implants useful in the present technology may be permanent tissue replacement devices, permanent stabilization devices, or temporary skeletal stabilization devices.

The orthopedic implants of the present technology include prosthetic implants or parts thereof. Joint replacement systems that may be coated include uncemented hips, knees, elbow, or shoulders. Orthopedic implants include uncemented devices that require tissue ingrowth or ongrowth to stabilize the implant, for example, for use in hip implants (e.g., femoral stems), knee implants (e.g., acetabular cups), elbow implants, shoulder implants, prosthetic frames, bone prostheses, and small joint prostheses. The devices to be coated can also include devices that do not require biologic fixation, such as fracture stabilization hardware (intramedullary nails, plates, screws), and arthrodesis hardware. Internal and external fixation implants and devices include bone plates, anchors, bone screws, rods, intramedullary nails, arthrodesis nails, pins, wires, spacers, and cages. The coating could also be applied to transdermal devices such as external fixation pins used in fracture stabilization or limb lengthening procedures. Such devices are commercially available from leading orthopedic device manufacturers, including Biomet Inc. (Warsaw, Indiana, USA). Other manufacturers include Zimmer, Inc. (Warsaw, IN, USA); DePuy Orthopedics, Inc. (Warsaw, Indiana, USA) and DePuy Spine, Inc. (Raynham, Massachusetts, USA).

The orthopedic implants of the present technology can comprise solid metals, for example, gold, silver, stainless steel, platinum, palladium, iridium, iron, nickel, copper, titanium, aluminum, chromium, cobalt, molybdenum, vanadium, tantalum, and alloys thereof. In preferred embodiments, the orthopedic implant comprises a metal including surgical stainless steel, titanium or a titanium alloy. In yet other embodiments, the orthopedic implant comprises a polymer, such as polyethylene, or a ceramic.

One or more surfaces of the implant, for example the surface to be coated with the infection-inhibiting coating, may be textured. The orthopedic implant surface to be coated with an infection-inhibiting coating can be textured uniformly with surface irregularities, including pores (micropores), dimples, spikes, ridges, grooves (e.g., microgrooves), roughened texture (e.g., microtextured), surface grain, strips, ribs, channels, ruts. The size of the micropores, dimples, spikes, ridges, grooves (e.g., microgrooves), roughened texture (e.g., microtextured), surface grain, strips, ribs, channels, ruts can range from 1 µm to 500 µm. In some embodiments, the size ranges from 10 µm to 100 µm. The texture may be formed by any suitable methods, for example, by molding, chemical etching, roughening with sandpaper or other abrasives (e.g., sand blasting and glass bead blasting), electrical means (such as EDM machining), thermal means, laser etching, or additive manufacturing processes.

In accordance with the present technology, the orthopedic implants can be coated with infection-inhibiting composition on at least one surface of the implant. Accordingly, the present technology provides methods for making an implantable medical device having an infection-inhibiting coating, the methods comprising rubbing an infection-inhibiting composition on a surface of the medical device, wherein a thin layer of the infection-inhibiting composition is coated on the surface, and wherein the infection-inhibiting composition comprises a waxy matrix comprising an infection-inhibiting material selected from the group consisting of a lipid, an antimicrobial agent, and mixtures thereof, wherein the waxy carrier is operable to deposit the infection-inhibiting material when rubbed on a surface of the device.

In some embodiments, all surfaces of the implant exposed to body tissues are coated. In other embodiments, the surfaces of the orthopedic implant to be coated with an infection-inhibiting composition are surfaces that are not intended to provide a structural network for tissue or cellular ingrowth or ongrowth. In some embodiments for coating of implants having articulating surfaces (e.g., hip implants), the infection-inhibiting composition is coated on an articulating surface of the implant.

In some embodiments, the waxy matrix dissipates from the medical device immediately after implantation. In particular, for use on orthopedic implants that are stabilized by biologic fixation or integration with host tissue, the lipid composition preferably dissolves from the implant surface very rapidly so as not to interfere with bone tissue ingrowth or ongrowth. The matrix may dissipate from the surface of the coated implant within hours after implantation. In some embodiments, the anti-infective is preferably released from the carrier over time, such as over the course of from 1 day to 3 weeks, or from 3 to 10 days.

The infection-inhibiting compositions can be applied in any appropriate manner, including application methods known to those of ordinary skill in the art of coated medical devices. For example, the infection-inhibiting composition can be rubbed or wiped onto the orthopedic implant. As discussed above, the composition can be applied using an infection-inhibiting delivery system of the present technology.

The materials and processes of the present technology are illustrated in the following non-limiting examples.

### Example 1

0.005 g of rifampin and 0.005 g of minocycline are dissolved in 0.05 g of ethanol to form an antimicrobial mixture. About half of the ethanol is allowed to evaporate to form a concentrated antimicrobial mixture, which is then stirred into 0.1 g of Phosal® 53 MCT (Lipoid Group, Köln, Germany) until a uniform antimicrobial and lecithin mixture is formed. The antimicrobial/lecithin mixture is then folded into 10 g of Phospholipon ® 90G (Lipoid Group, Köln, Germany) until a uniform composition is formed. The final composition contains:
0.1% each of rifampin and minocycline;
0.5% ethanol;
1% of a mixture of 50% phosphatidylcholine and 50% various other lipids; and
98.4% of a mixture of at least 90% phosphatidyl choline and the remainder a mixture of various other smaller lipids.

### Example 2

Formulation "90G" was made consisting entirely of Phospholipon 90G purified soy phosphatidylcholine, with a minimum purity of 94% phosphatidylcholine by weight. The yellowish, waxy solid material is supplied as small clumps. To form the material into a stick-form composition, it was repeatedly ground in a ceramic mortar and pestle that was heated to 40 °C, then kneaded until solid, and then 4 gram aliquots were cold pressed into a 12 mm diameter cylinder.

### Example 3

Formulation "90G90H" was made by grinding together 6 grams of Phospholipon 90G and 3 grams of Phospholipon 90H (Lipoid Group, Köln, Germany). Phospholipon 90H is white powder purified soy derived phosphatidylcholine that is hydrogenated (fully saturated). The Phospholipon 90H was blended with the unsaturated natural phosphatidylcholine, and preheated to 60 °C to soften the hydrogenated form. The two materials were finely ground together in a ceramic mortar and pestle that was heated to 40 °C. The mixture was kneaded until a smooth, solid, and cohesive waxy solid was created. Four gram aliquots were cold molded into 12 mm diameter cylindrical sticks.

### Example 4

Formulation "90G53MCT" was made by combining 3 grams of Phospholipon 90G with 1 gram of Phosal 53MCT (Lipoid GmbH, Köln, Germany). Phosal 53MCT is a mixture of lipids, comprising at least 53% phosphatidylcholine, dissolved in medium chain triglycerides and sunflower oil. Phosal 53MCT is liquid at room temperature. A mixture of Phosal 53MCT and Phospholipon 90G was made by finely grinding together in a ceramic mortar and pestle that was heated to 40 °C , then kneading until a smooth, solid, and cohesive waxy is solid formed. The waxy solid was cold molded into a 12 mm diameter cylindrical stick.

### Example 5

Formulation "10%R90G" was made by grinding together 0.8 grams of the antibiotic rifampin (Lupin Pharmaceuticals, Mumbai, India), and 7.2 grams of Phospholipon 90G in a ceramic mortar and pestle that was heated to 40 °C, then kneading until a smooth, solid, and cohesive waxy solid is formed. 4 gram aliquots were cold molded into 12 mm diameter cylindrical sticks.

### Example 6

Formulation "25%R90G" was made by combining 2 grams rifampin and 6 grams Phospholipon 90G and grinding, kneading, and molding into cylindrical sticks as per Example 5, above.

### Example 7

Formulation "10%R90G90H" was made by grinding together 0.8 grams of the antibiotic rifampin (Lupin Pharmaceuticals, Mumbai, India), and 7.2 grams of the 90G90H carrier from formulation "90G90H" in a ceramic mortar and pestle that was heated to 40 °C, then kneaded until a smooth, solid, and cohesive waxy solid is formed. 4 gram aliquots were cold molded into 12 mm diameter cylindrical sticks.

### Example 8

Formulation "25%R90G90H" was made by combining 2 grams rifampin and 6 grams of 90G90H and mixing and molding into cylindrical sticks as in Example 7, above.

### Example 9

Formulation "10%R90G53MCT" was made by mixing together 0.8 g rifampin and 1.8 grams of Phosal 53MCT, then grinding together with 5.4 grams of Phospholipon 90G to form a mixture. The mixture was finely ground in a ceramic mortar and pestle that is heated to 40 °C, and then kneaded until a smooth, solid, and cohesive waxy solid was formed. 4 gram aliquots of the waxy solid were then cold molded into 12 mm diameter cylindrical sticks.

### Example 10 (not according to the present invention)

Formulation "10%V90G" was made by grinding a 0.8 grams of the antibiotic vancomycin (Axellia Pharmaceuticals, Oslo, Norway), and 7.2 grams of Phospholipon 90G in a ceramic mortar and pestle that was heated to 40 °C. The mixture was then kneaded until a smooth, solid, and cohesive waxy solid formed. 4 gram aliquots of the waxy solid were then cold molded into 12 mm diameter cylindrical sticks.

### Example 11 (not according to the present invention)

Formulation "25%V90G" was made by combining 2 grams vancomycin and 6 grams Phospholipon 90G and grinding, kneading, and molding into cylindrical sticks in Example 10, above.

### Example 12

Formulation "10%PLY90G" was made by grinding 0.8 grams of the food preservative, epsilon polylysine (Zhengahou Bainfo Bioengineering company, China) and 7.2 grams of Phospholipon 90G in a ceramic mortar and pestle that is heated to 40 °C. The mixture was kneaded until a smooth, solid, and cohesive waxy solid was formed. 4 gram aliquots of the waxy solid were cold molded into 12 mm diameter cylindrical sticks.

### Example 13

Gentamicin sulfate powder was mixed with Phospholipon 90G by finely grinding in a ceramic mortar and pestle. The mixture was kneaded until a smooth, uniform, cohesive solid was obtained. The solid was then molded into a 12 mm diameter cylindrical sticks. Two formulations were mixed and molded: 10% gentamicin sulfate by weight, and 25% gentamicin sulfate by weight.

### Testing

The compositions of Examples 2 - 12, summarized in Table 1 below, were tested for hardness and density of coating when applied to a surface, and coating performance.

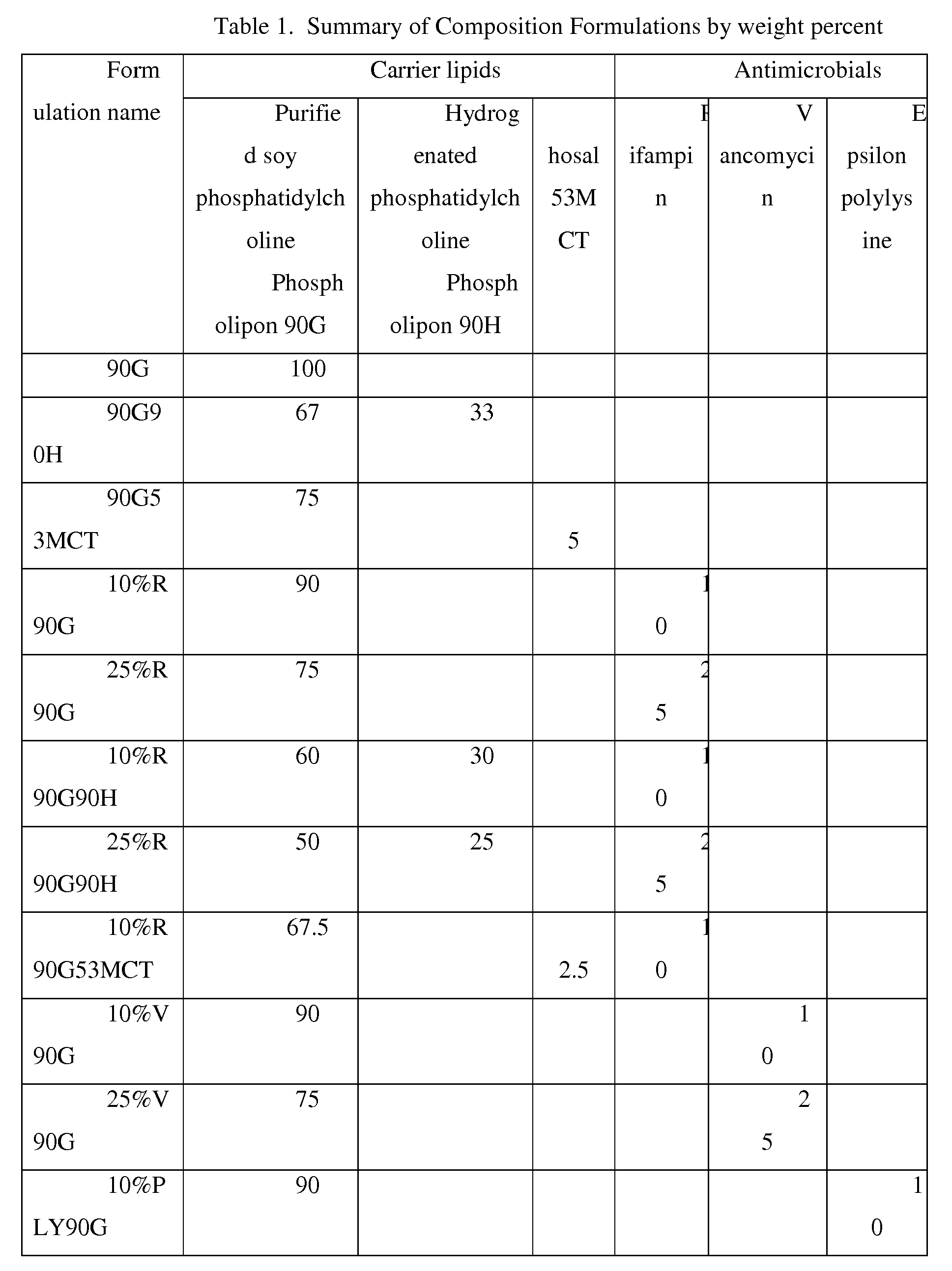

### Mechanical Testing

12 mm diameter unconstrained cylinders 12 mm tall of each of the 11 formulations of Examples 2 - 12 were tested for hardness by cone penetration, using a cone having an about a 27.5 degree angle that widened to a 6.25 mm diameter over a 6 mm distance. The cone was lowered into the 12 mm diameter 12 mm tall cylinder of the composition material at a rate of 1 mm per second for a distance of 5 mm. A ten pound (44.48 N) load cell was used to measure the hardness as the peak load experienced by the cone over the 5 mm distance of travel into the sample. This test is a modification of the ASTM standard test method D1321 for needle penetration of petroleum waxes. The results of the mechanical testing are shown in Table 2. All the formulations had a hardness of between 1.9 lbf (8.45 N) to 10.2 lbf (45.37 N).

**Table 2. Mechanical testing results.**

| Formulation name | Hardness lbf (N) |
|---|---|
| 90G | 3.4 (15.12) |
| 90G90H | 7.9 (35.14) |
| 90G53MCT | 1.9 (8.45) |
| 10%R90G | 3.6 (16.01) |
| 25%R90G | 6.3 (28.02) |
| 10%R90G90H | 4.9 (21.8) |
| 25%R90G90H | 5.6 (24.91) |
| 10%R90G53MCT | 2.2 (9.79) |
| 10%V90G | 8.2 (36.47) |
| 25%V90G | 10.2 (45.37) |
| 10%PLY90G | 5.9 (26.24) |

### Coating Density and Antimicrobial Dose

The infection-inhibiting compositions of Examples 2 - 12 were rubbed onto tared coupons of both smooth-finished and rough-finished (30-grit blasted) titanium alloy. The weight of the coating that transferred was recorded for each formulation. An aqueous saline solution was tested in lieu of a concentrated solution of antibiotic. Typical concentration of an antibiotic solution for IV use (prior to further dilution in the injection solution) was 50 mg per ml of water. The amount of antibiotic per cm² of surface area was calculated based on the weight of the coating and the concentration in the infection-inhibiting composition, or for the antibiotic solution, was approximated by the volume of water that adhered to the surface. Table 3 shows the coating densities applied onto the coupons.

**Table 3. Coating Densities Applied to Coupons**

| Formulation name | Coating Weight on Smooth Surface (mg/cm²) | Coating Weight on Rough Surface (mg/cm²) | Antimic robial Agent Dose on Smooth Surface (µg/cm²) | Antimic robial Agent Dose on Rough Surface (µg/cm²) |
|---|---|---|---|---|
| 90G | 0.99 | 2.9 | - | - |
| 90G90H | 0.28 | 1.7 | - | - |
| 90G53MCT | 0.28 | 3.2 | - | - |
| 10%R90G | 0.21 | 2.3 | 21 | 230 |
| 25%R90G | 0.18 | 1.8 | 44 | 450 |
| 10%R90G90H | 0.11 | 1.9 | 11 | 190 |
| 25%R90G90H | 0.14 | 1.7 | 35 | 425 |
| 10%R90G53MCT | 0.42 | 3 | 42 | 300 |
| 10%V90G | 0.60 | 2.2 | 60 | 220 |
| 25%V90G | 0.25 | 4.1 | 62 | 1025 |
| 10%PLY90G | 0.46 | 1.9 | 46 | 190 |
| Antibiotic Solution | 0.04 | 1 | 2 | 50 |

As used herein, the words "preferred" and "preferably" refer to embodiments of the technology that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the technology.

As referred to herein, all compositional percentages are by weight of the total composition, unless otherwise specified. As used herein, the word "include," and its variants, is intended to be non-limiting, such that recitation of items in a list is not to the exclusion of other like items that may also be useful in the materials, compositions, devices, and methods of this technology. Similarly, the terms "can" and "may" and their variants are intended to be non-limiting, such that recitation that an embodiment can or may comprise certain elements or features does not exclude other embodiments of the present technology that do not contain those elements or features.

As used herein, the term "operable" refers to a material, device or action which is capable, by virtue of its composition, design or features, to perform a recited function. In some embodiments, an operable material device or action is adapted to perform the function, having a specific composition, design or feature that is adapted (relative to similar composition, design or features known in the art), individually or in combination with other composition, design and features of the present technology, for use in performing the recited function. An operable material, device or action may, in some embodiments, also be capable of performing other functions.

## Claims

1. An infection-inhibiting composition for application to the surface of an implantable medical device, comprising an antimicrobial agent in a waxy matrix comprising phosphatidylcholine, wherein the matrix is operable to deposit the anti-infective when rubbed on a surface of the device.

2. The infection-inhibiting composition according to Claim 1, wherein the waxy matrix comprises 90% or more, by weight of the matrix, of phosphatidylcholine.

3. The infection-inhibiting composition according to Claim 1, wherein the antimicrobial agent is present at a level of from 0.1% to 35% by weight.

4. The infection-inhibiting composition according to Claim 1, wherein the antimicrobial agent is selected from the group consisting of antibiotics, antimicrobial peptides, disinfectants, and antimicrobial metal ions, epsilon polylysine, sugar alcohols, essential oils, and mixtures thereof.

5. The infection-inhibiting composition according to Claim 4, wherein the antimicrobial agent is selected from the group consisting of fosfomycin, rifampin, tetracyclines, aminoglycosides, quinolones, glycopeptides, and mixtures thereof, preferably comprising rifampin and minocycline.

6. The infection-inhibiting composition according to Claim 1, having a cone penetration test hardness of at least 1.5 lbf (6.67 N), preferably from 5 lbf (22.24 N) to 15 lbf (66.7 N).

7. An orthopedic device for inhibiting infection at the site of implantation of the orthopedic device, preferably having a textured surface, in a human or animal subject, obtained by a method comprising rubbing a surface of the device, prior to implantation, with an infection-inhibiting composition comprising phosphatidylcholine and having a waxy matrix comprising an infection-inhibiting material selected from the group consisting of a lipid, an antimicrobial agent, and mixtures thereof, wherein a thin layer of the infection-inhibiting material is deposited on the surface of the device.

8. The orthopedic device for inhibiting infection according to Claim 7, wherein the waxy matrix comprises a lipid selected from the group consisting of fatty acids, triacylglyerols, diacylglycerols, glycerophospholipids, and mixtures thereof..

9. The orthopedic device for inhibiting infection according to Claim 8, wherein the composition wherein the waxy matrix comprises 90% or more, by weight of the matrix, of phosphatidylcholine.

10. The orthopedic device for inhibiting infection according to Claim 7, comprising an antimicrobial agent selected from the group consisting of antibiotics, antimicrobial peptides, disinfectants, and antimicrobial metal ions, epsilon polylysine, sugar alcohols, essential oils, and mixtures thereof, preferably selected from the group consisting of fosfomycin, rifampin, tetracyclines, aminoglycosides, quinolones, glycopeptides, and mixtures thereof.

11. The orthopedic device for inhibiting infection according to Claim 7, wherein the composition has a cone penetration test hardness from 5 lbf (22.24 N) to 15 lbf (66.7 N).

12. The orthopedic device for inhibiting infection according to Claim 9, wherein the orthopedic device is a hip implant, a knee implant, an elbow implant, a prosthetic frame, bone plate, a bone prosthesis, a small joint prosthesis, a rod, a pin, a hook, an intramedullary nail or other nail, a bone screw, a spacer or a cage.

13. The orthopedic device for inhibiting infection according to Claim 7, wherein the sterile medical device is coated with the infection-inhibiting composition in the operating room prior to implantation in a patient.

## Patentansprüche

1. Infektionshemmende Zusammensetzung zum Auftragen auf die Oberfläche einer implantierbaren medizinischen Vorrichtung, die ein antimikrobielles Mittel in einer wachsartigen Matrix umfasst, die Phosphatidylcholin umfasst, wobei die Matrix dazu dient, um das Antiinfektivum abzuscheiden, wenn sie auf eine Oberfläche der Vorrichtung gerieben wird.

2. Infektionshemmende Zusammensetzung nach Anspruch 1, wobei die wachsartige Matrix 90 % oder mehr Phosphatidylcholin bezogen auf das Gewicht der Matrix umfasst.

3. Infektionshemmende Zusammensetzung nach Anspruch 1, wobei das antimikrobielle Mittel in einer Menge von 0,1 bis 35 Gew.-% vorhanden ist.

4. Infektionshemmende Zusammensetzung nach Anspruch 1, wobei das antimikrobielle Mittel aus der Gruppe ausgewählt ist, die aus Antibiotika, antimikrobiellen Peptiden, Desinfektionsmitteln und antimikrobiellen Metallionen, Epsilonpolylysin, Zuckeralkoholen, ätherischen Ölen und Mischungen davon besteht.

5. Infektionshemmende Zusammensetzung nach Anspruch 4, wobei das antimikrobielle Mittel aus der Gruppe ausgewählt ist, die aus Fosfomycin, Rifampin, Tetracyclinen, Aminoglykosiden, Chinolonen, Glykopeptiden und Mischungen davon besteht und vorzugsweise Rifampin und Minocyclin umfasst.

6. Infektionshemmende Zusammensetzung nach Anspruch 1 mit einer Kegeleindringtesthärte von mindestens 1,5 lbf (6,67 N), vorzugsweise von 5 lbf. (22,24 N) bis 15 lbf (66,7 N).

7. Orthopädische Vorrichtung zum Hemmen einer Infektion am Ort der Implantation einer orthopädischen Vorrichtung, vorzugsweise mit einer strukturierten Oberfläche, in einem Menschen oder Tier, die durch ein Verfahren erhalten wird, dass ein Reiben einer Oberfläche der Vorrichtung vor der Implantation mit einer infektionshemmenden Zusammensetzung umfasst, die Phosphatidylcholin umfasst und eine wachsartige Matrix aufweist, die ein infektionshemmendes Material umfasst, das aus der Gruppe ausgewählt ist, die aus einem Lipid, einem antimikrobiellen Mittel und Mischungen davon besteht, wobei eine dünne Schicht des infektionshemmenden Materials auf der Oberfläche der Vorrichtung abgeschieden wird.

8. Orthopädische Vorrichtung zum Hemmen einer Infektion nach Anspruch 7, wobei die wachsartige Matrix ein Lipid umfasst, das aus der Gruppe ausgewählt ist, die aus Fettsäuren, Triacylglyerolen, Diacylglycerinen, Glycerophospholipiden und Mischungen davon besteht.

9. Orthopädische Vorrichtung zum Hemmen einer Infektion nach Anspruch 8, wobei die Zusammensetzung wobei die wachsartige Matrix 90 % oder mehr Phosphatidylcholin bezogen auf das Gewicht der Matrix umfasst.

10. Orthopädische Vorrichtung zum Hemmen einer Infektion nach Anspruch 7, umfassend ein antimikrobielles Mittel, das aus der Gruppe ausgewählt ist, die aus Antibiotika, antimikrobiellen Peptiden, Desinfektionsmitteln und antimikrobiellen Metallionen, Epsilonpolylysin, Zuckeralkoholen, ätherischen Ölen und Mischungen derselben besteht, und vorzugsweise aus der Gruppe ausgewählt ist, die aus Fosfomycin, Rifampin, Tetracyclinen, Aminoglykosiden, Chinolonen, Glykopeptiden und Mischungen davon besteht.

11. Orthopädische Vorrichtung zum Hemmen einer Infektion nach Anspruch 7, wobei eine Zusammensetzung eine Kegeleindringtesthärte von 5 lbf (22,24 N) bis 15 lbf (66,7 N) aufweist.

12. Orthopädische Vorrichtung zum Hemmen einer Infektion nach Anspruch 9, wobei die orthopädische Vorrichtung ein Hüftimplantat, ein Knieimplantat, ein Ellbogenimplantat, ein Prothesenrahmen, eine Knochenplatte, eine Knochenprothese, eine kleine Gelenkprothese, eine Stange, ein Stift, ein Haken, ein Marknagel oder ein anderer Nagel, eine Knochenschraube, ein Distanzstück oder ein Käfig ist.

13. Orthopädische Vorrichtung zum Hemmen einer Infektion nach Anspruch 7, wobei die sterile medizinische Vorrichtung vor der Implantation in einen Patienten im Operationssaal mit der infektionshemmenden Zusammensetzung beschichtet ist.

## Revendications

1. Composition d'inhibition d'infection pour application à la surface d'un dispositif médical implantable, comprenant un agent antimicrobien dans une matrice cireuse comprenant de la phoshatidylcholine, où la matrice peut fonctionner pour déposer l'anti-infectieux quand elle est frottée sur une surface du dispositif.

2. Composition d'inhibition d'infection selon la revendication 1, dans laquelle la composition cireuse comprend 90 % ou plus, en poids de la matrice, de phosphatidylcholine.

3. Composition d'inhibition d'infection selon la revendication 1, dans laquelle l'agent anti-microbien est présent à un niveau de 0,1 % à 35 % en poids.

4. Composition d'inhibition d'infection selon la revendication 1, dans laquelle l'agent anti-microbien est choisi dans le groupe constitué par les antibiotiques, les peptides antimicrobiens, les désinfectants et les ions métalliques antimicrobiens, l'epsilon polylysine, les alcools sucrés, les huiles essentielles et leurs mélanges.

5. Composition d'inhibition d'infection selon la revendication 4, dans laquelle l'agent anti-microbien est choisi dans le groupe constitué par la fosfomycine, la rifampine, les tétracyclines, les aminoglycosides, les quinolones, les glycopeptides et leurs mélanges, de préférence comprenant la rifampine et la minocycline.

6. Composition d'inhibition d'infection selon la revendication 1, ayant une dureté de test de pénétration de cône d'au moins 1,5 lbf (6,67 N), de préférence de 5 lbf (22,24 N) à 15 lbf (66,7 N).

7. Dispositif orthopédique pour inhiber une infection au site d'implantation du dispositif orthopédique, de préférence ayant une surface texturée, chez un sujet humain ou animal, obtenu par un procédé comprenant le frottement d'une surface du dispositif, avant l'implantation, avec une composition d'inhibition d'infection comprenant de la phosphatidylcholine et ayant une matrice cireuse comprenant un matériau d'inhibition d'infection choisi dans le groupe constitué par un lipide, un agent anti-microbien et leurs mélanges, où une couche mince du matériau inhibant l'infection est déposée sur la surface du dispositif.

8. Dispositif orthopédique pour inhiber une infection selon la revendication 7, dans lequel la matrice cireuse comprend un lipide choisi dans le groupe constitué par les acides gras, les triacylglycérols, les diacylglycérols, les glycérophopsholipides et leurs mélanges.

9. Dispositif orthopédique pour inhiber une infection selon la revendication 8, dans lequel la composition où la matrice cireuse comprend 90 % ou plus, en poids de matrice, de phosphatidylcholine.

10. Dispositif orthopédique pour inhiber une infection selon la revendication 7, comprenant un agent antimicrobien choisi dans le groupe constitué par les antibiotiques, les peptides antimicrobiens, les désinfectants et les ions métalliques antimicrobiens, l'epsilon polylysine, les alcools sucrés, les huiles essentielles et leurs mélanges, de préférence choisi dans le groupe constitué par la fosfomycine, la rifampine, les tétracyclines, les aminoglycosides, les quinolones, les glycopeptides et leurs mélanges.

11. Dispositif orthopédique pour inhiber une infection selon la revendication 7, dans lequel la composition a une dureté de test de pénétration de cône de 5 lbf (22,24 N) à 15 lbf (66,7 N).

12. Dispositif orthopédique pour inhiber une infection selon la revendication 9, dans lequel le dispositif orthopédiques est un implant de hanche, un implant de genou, un implant de coude, un cadre prosthétique, une plaque d'os, une prothèse d'os, une prothèse de petite articulation, une tige, une broche, un crochet, un ongle intramédullaire ou un autre ongle, une vis d'os, un espaceur ou une cage.

13. Dispositif orthopédique pour inhiber une infection selon la revendication 7, dans lequel le dispositif médical stérile est revêtu avec une composition inhibant une infection dans la chambre d'opération avant l'implantation chez un patient.
